(19) Europäisches Patentamt
European Patent Office
Office européen des brevets

(11) Veröffentlichungsnummer: **0 093 908**
**A1**

(12) # EUROPÄISCHE PATENTANMELDUNG

(21) Anmeldenummer: **83103892.2**

(22) Anmeldetag: **21.04.83**

(51) Int. Cl.³: **C 07 D 213/70**, C 07 D 277/74, C 07 D 239/38, C 07 D 215/36, C 07 D 233/84, C 07 D 235/28, C 07 D 263/58, A 01 N 43/00

(30) Priorität: **03.05.82 DE 3216416**

(43) Veröffentlichungstag der Anmeldung: **16.11.83**
**Patentblatt 83/46**

(84) Benannte Vertragsstaaten: **BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Heinemann, Ulrich, Dr., Thorner Strasse 22, D-5600 Wuppertal 2 (DE)**
Erfinder: **Frohberger, Paul-Ernst, Dr., Willi-Baumeister-Strasse 5, D-5090 Leverkusen 1 (DE)**
Erfinder: **Brandes, Wilhelm, Dr., Eichendorffstrasse 3, D-5653 Leichlingen (DE)**

(54) **Heteroarylthiovinyl-Verbindungen, ein Verfahren zu ihrer Herstellung und ihre Verwendung als Schädlingsbekämpfungsmittel.**

(57) Man erhält die neuen Heteroarylthiovinyl-Verbindungen der Formel (I)

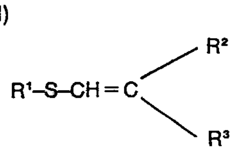

(I)

in welcher

$R^1$ für einen gegebenenfalls substituierten heteroaromatischen Rest steht und

$R^2$ und $R^3$ gleich oder verschieden sind und für Cyano, Nitro, Alkoxycarbonyl, Alkylsulfonyl oder für gegebenenfalls substituiertes Phenylsulfonyl stehen,

durch Umsetzung von substituierten Vinylhalogeniden der Formel (II)

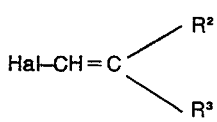

(II)

mit Heteroarylthiolen der Formel (III)

$$R^1\text{–}SH \qquad (III)$$

in welchen

$R^1$–$R^3$ die angegebenen Bedeutungen haben und

Hal für Halogen steht,

in Gegenwart eines organischen Verdünnungsmittels und in Gegenwart einer Base.

Die Verbindungen der Formel (I) können vor allem als Fungizide verwendet werden.

EP 0 093 908 A1

BAYER AKTIENGESELLSCHAFT 5090 Leverkusen, Bayerwerk

Zentralbereich
Patente, Marken und Lizenzen Bas/by-c
Ia

# Heteroarylthiovinyl-Verbindungen, ein Verfahren zu ihrer Herstellung und ihre Verwendung als Schädlingsbekämpfungsmittel

Die vorliegende Erfindung betrifft neue Heteroarylthiovinyl-Verbindungen, ein Verfahren zu ihrer Herstellung und ihre Verwendung als Schädlingsbekämpfungsmittel, insbesondere als Fungizide.

Es ist bereits bekannt geworden, daß organische Schwefelverbindungen, wie z.B. Zink-ethylen-1,2-bis-(dithiocarbamat), gute fungizide Eigenschaften aufweisen (vgl. R. Wegler "Chemie der Pflanzenschutz- und Schädlingsbekämpfungsmittel", Springer Verlag Berlin 1970, Bd. 2, S. 65 f). Die Wirkung dieser Verbindungen ist jedoch insbesondere bei niedrigen Aufwandmengen und -konzentrationen, in einigen Anwendungsbereichen nicht immer voll befriedigend.

Es wurden neue Heteroarylthiovinyl-Verbindungen der allgemeinen Formel (I)

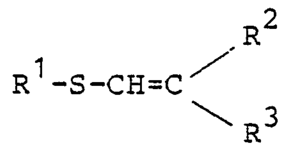

(I)

in welcher

$R^1$ für einen gegebenenfalls substituierten heteroaromatischen Rest steht und

$R^2$ und $R^3$ gleich oder verschieden sind und für Cyano, Nitro, Alkoxycarbonyl, Alkylsulfonyl oder gegebenenfalls substituiertes Phenylsulfonyl stehen,

gefunden.

Die Verbindungen der Formel (I) können in verschiedenen geometrischen Isomerenformen vorliegen, je nach Anordnung der Gruppen an den Kohlenstoffatomen der Vinyldoppelbindung. Sowohl die Isomeren als auch deren Gemische werden erfindungsgemäß beansprucht.

Weiterhin wurde gefunden, daß man die Heteroarylthiovinyl-Verbindungen der allgemeinen Formel (I) erhält, wenn man substituierte Vinylhalogenide der allgemeinen Formel (II)

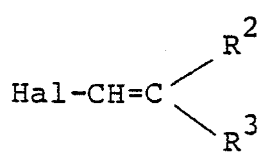

(II)

in welcher

$R^2$ und $R^3$ gleich oder verschieden sind und für Cyano, Nitro, Alkoxycarbonyl, Alkylsulfonyl oder für gegebenenfalls substituiertes Phenylsulfonyl und

Hal für Halogen stehen,

mit Heteroarylthiolen der Formel (III)

$$R^1\text{-SH} \qquad \text{(III)}$$

in welcher

$R^1$ für einen gegebenenfalls substituierten heteroaromatischen Rest steht,

in Gegenwart eines organischen Verdünnungsmittels und in Gegenwart einer Base umsetzt.

Die neuen Heteroarylthiovinyl-Verbindungen der Formel (I) weisen starke fungizide Eigenschaften auf. Dabei zeigen die erfindungsgemäßen Verbindungen der Formel (I) überraschenderweise eine bessere fungizide Wirksamkeit auf als das nach dem Stand der Technik bekannte Zink-ethylen-1,2-bis(dithiocarbamat).

Die erfindungsgemäßen Stoffe stellen somit eine Bereicherung der Technik dar.

Die erfindungsgemäßen Heteroarylthiovinyl-Verbindungen sind durch die Formel (I) allgemein definiert. In dieser Formel stehen vorzugsweise

$R^1$ für einen gegebenenfalls ein- oder zweifach, gleich oder verschieden substituierten über Kohlenstoff verknüpften heteroaromatischen 5- oder 6-gliedrigen Ring, der auch benzoanelliert sein kann. Als Substituenten kommen vorzugsweise Alkyl mit 1 bis 4 Kohlenstoffatomen, Halogen und Nitro in Frage, und

$R^2$ und $R^3$, welche gleich oder verschieden sind für Cyano, Nitro, Alkoxycarbonyl oder Alkylsulfonyl mit jeweils 1 bis 4 Kohlenstoffatomen im Alkylteil oder für gegebenenfalls ein- oder zweifach, gleich oder verschieden substituiertes Phenylsulfonyl . Als Substituenten seien vorzugsweise Alkyl mit 1 bis 4 Kohlenstoffatomen und Halogen genannt.

Besonders bevorzugt sind diejenigen Verbindungen der Formel (I), in denen

$R^1$ für über Kohlenstoff verknüpftes, gegebenenfalls ein- oder zweifach, gleich oder verschieden substituiertes Pyridyl, Pyrimidyl, Imidazolyl, Chinolyl, Benzothiazolyl, Benzimidazolyl und Benzoxazolyl steht, wobei als Substituenten vorzugsweise genannt seien: Methyl, Ethyl, n-Propyl, iso-Propyl, n-, iso-, sec- und t-Butyl, Chlor, Brom und Nitro.

$R^2$ und $R^3$ gleich oder verschieden sind und für Cyano, Nitro, Methoxycarbonyl, Ethoxycarbonyl, Methylsulfonyl, Ethylsulfonyl und für gegebenenfalls ein- oder zweifach, gleich oder verschieden substituiertes Phenylsulfonyl stehen, wobei als Substituenten besonders bevorzugt Methyl, Ethyl, Chlor und Brom sind.

Im einzelnen sei auf die bei den Herstellungsbeispielen genannten Verbindungen verwiesen.

Verwendet man beispielsweise 2-Mercaptopyridin und 1-Chlor-2,2-dicyanoethen als Ausgangsstoffe, so kann der Reaktionsablauf des erfindungsgemäßen Verfahrens durch das folgende Formelschema wiedergegeben werden:

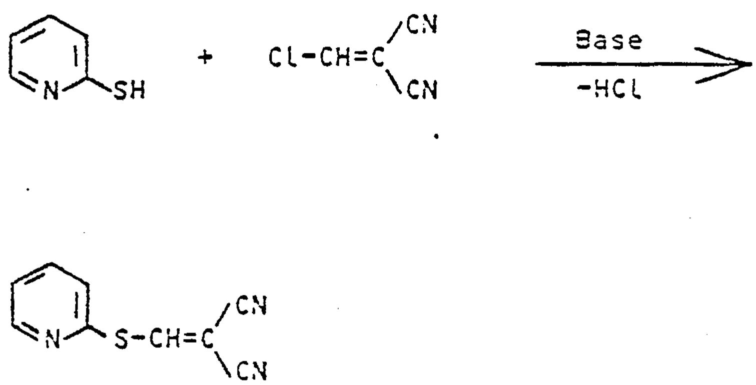


Die bei der Durchführung des erfindungsgemäßen Verfahrens als Ausgangsstoffe zu verwendenden substituierten Vinylhalogenide sind durch die Formel (II) allgemein definiert. In dieser Formel stehen

$R^2$ und $R^3$ vorzugsweise für die Reste, die bereits im Zusammenhang mit der Beschreibung der erfindungsgemäßen Stoffe der Formel (I) vorzugsweise für diese Substituenten genannt werden.

Hal steht vorzugsweise für Chlor oder Brom.

Die substituierten Vinylhalogenide der Formel (II) sind bekannt (vgl. z.B. J. Org. Chemistry 32, 1941-1944 (1967)) oder können nach dem dort beschriebenen Verfahren in analoger Weise hergestellt werden.

Die außerdem bei der Durchführung des erfindungsgemäßen Verfahrens als Ausgangsstoffe zu verwendenden Heteroarylthiole sind durch die Formel (III) allgemein definiert. In dieser Formel steht $R^1$ vorzugsweise für die Reste, die bereits im Zusammenhang mit der Beschreibung der erfindungsgemäßen Stoffe der Formel (I) vorzugsweise für diesen Substituenten genannt wurden.

Die Heteroarylthiole der Formel (III) sind allgemein bekannte Verbindungen der organischen Chemie (vgl. z.B. S. Patai "The chemistry of the thiol group", Part 1, John Wiley and Sons, New York 1974, S. 182).

Für die erfindungsgemäße Umsetzung kommen als Verdünnungsmittel inerte organische Lösungsmittel infrage. Hierzu gehören vorzugsweise aromatische

Kohlenwasserstoffe wie Benzol, Toluol oder Xylol, halogenierte Kohlenwasserstoffe wie Methylenchlorid, Chloroform, Tetrachlorkohlenstoff oder Chlorbenzol, Ketone wie Aceton oder Butanon, Ether wie Diethylether, Dioxan, Tetrahydrofuran, Formamide wie Dimethylformamid, Nitrile wie Acetonitril oder Propionitril, sowie die hochpolaren Lösungsmittel Dimethylsulfoxid oder Hexamethylphosphorsäuretriamid.

Das erfindungsgemäße Verfahren wird in Gegenwart einer Base durchgeführt. Hierbei können alle üblichen organischen und insbesondere anorganischen Basen eingesetzt werden. Vorzugsweise verwendet man Alkali- oder Erdalkalicarbonate, beispielsweise seien Natrium- oder Kaliumcarbonat genannt.

Die Reaktionstemperaturen können bei der Durchführung des erfindungsgemäßen Verfahrens in einem größeren Bereich variiert werden. Im allgemeinen arbeitet man zwischen -20°C und +100°C, vorzugsweise zwischen 0°C und 70°C. Das erfindungsgemäße Verfahren wird im allgemeinen bei Normaldruck durchgeführt.

Bei der Durchführung des erfindungsgemäßen Verfahrens arbeitet man vorzugsweise in äquimolaren Mengen. Die Isolierung der Endprodukte der Formel (I) erfolgt in allgemein üblicher Art und Weise.

Die erfindungsgemäßen Wirkstoffe weisen eine starke mikrobizide Wirkung auf und können zur Bekämpfung von

0093908

unerwünschten Mikroorganismen praktisch eingesetzt werden. Die Wirkstoffe sind für den Gebrauch als Pflanzenschutzmittel geeignet.

So werden z.B. fungizide Mittel im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Wirkstoffe mit besonders gutem Erfolg zur Bekämpfung von Venturia-Arten, wie z.B. gegen den Erreger des Apfelschorfs (Venturia inaequalis) oder zur Bekämpfung von Erysiphe-Arten, wie z.B. den Erreger des echten Getreidemehltaus (Erysiphe graminis) sowie zur Bekämpfung von Reiskrankheiten, wie z.B. Pyricularia oryzae, eingesetzt werden.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und

Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Struen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

## Herstellungsbeispiele

### Beispiel 1

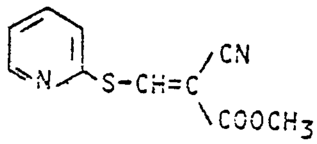

Zu 9,0 g (0,083 Mol) 2-Mercaptopyridin und 22,5 g (0,166 Mol) Kaliumcarbonat in 100 ml N,N-Dimethylformamid werden 12,0 g (0,083 Mol) ß-Chlor-α-cyano-acrylsäuremethylester zugegeben. Es wird 10 Minuten auf 70°C erwärmt und dann bei 20-25°C noch zwei Stunden nachgerührt. Nach Zugabe von 250 ml Wasser werden die ausgefallenen Kristalle abgesaugt und getrocknet. Man erhält 13,0 g (72 % der Theorie) 1-(2-Pyridylthio)-2-cyano-2-methoxy-carbonylethen vom Schmelzpunkt 153-154°C.

### Beispiel 2

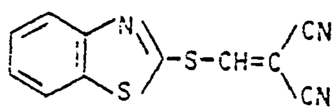

Zu 16,7 g (0,1 Mol) 2-Mercaptobenzothiazol und 13,8 g (0,1 Mol) Kaliumcarbonat in 150 ml Acetonitril werden 11,2 g (0,1 Mol) ß-Chlor-α-cyano-acrylnitril bei 5°C zugetropft. Die Mischung wird 2 Stunden bei 5-10°C gerührt. Dann werden 300 ml Wasser zugegeben und die ausgefallenen Kristalle abgesaugt. Man erhält 20,8 g

(86 % der Theorie) 1-(2-Benzthiazolylthio)-2,2-dicyano-ethen vom Schmelzpunkt 110°C (Zers.).

In entsprechender Weise werden die Verbindungen der allgemeinen Formel (I) erhalten:

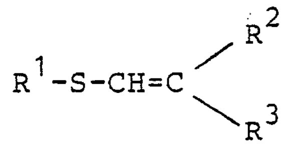

(I)

| Beisp. Nr. | $R^1$ | $R^2$ | $R^3$ | Schmelzpunkt (°C) |
|---|---|---|---|---|
| 3 | 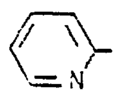 | CN | CN | 100 |
| 4 | 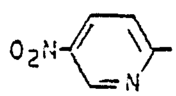 | CN | $COOCH_3$ | 170 |
| 5 | 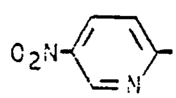 | CN | CN | 12o - 125 |
| 6 | 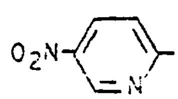 | $COOCH_3$ | $COOCH_3$ | 185 |
| 7 | 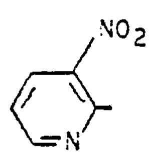 | $COOCH_3$ | $COOCH_3$ | 145 - 147 |
| 8 | 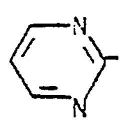 | CN | $COOCH_3$ | 141 |
| 9 | 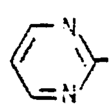 | CN | CN | 145 |

| Beisp. Nr. | $R^1$ | $R^2$ | $R^3$ | Schmelzpunkt (°C) |
|---|---|---|---|---|
| 10 | 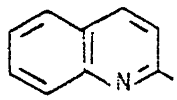 | CN | $COOCH_3$ | 149 (Zers.) |
| 11 | 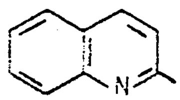 | CN | CN | 127-130 (Zers.) |
| 12 | 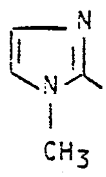 | CN | $COOCH_3$ | 210 |
| 13 | 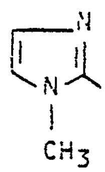 | CN | CN | 210-213 (Zers.) |
| 14 | 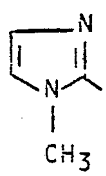 | $COOCH_3$ | $COOCH_3$ | 140-142 |
| 15 | 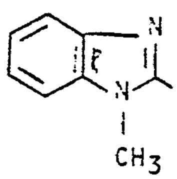 | CN | $COOCH_3$ | 130-135 |
| 16 | 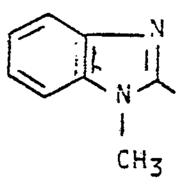 | $COOCH_3$ | $COOCH_3$ | 174-175 |

| Beisp. Nr. | $R^1$ | $R^2$ | $R^3$ | Schmelzpunkt (°C) |
|---|---|---|---|---|
| 17 | 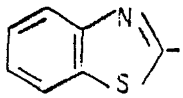 | CN | $COOCH_3$ | 115 (Zers.) |
| 18 | 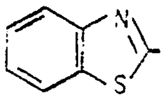 | $COOCH_3$ | $COOCH_3$ | 120-123 (Zers.) |
| 19 | 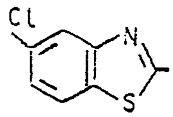 | CN | $COOCH_3$ | 148-150 (Zers.) |
| 20 | 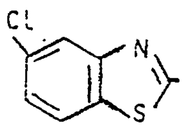 | $COOCH_3$ | $COOCH_3$ | 128-130 |
| 21 | 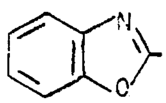 | CN | $COOCH_3$ | 138-140 |

## Anwendungsbeispiele

In den nachfolgenden Beispielen wird die nachstehend angegebene Verbindung als Vergleichssubstanz eingesetzt:

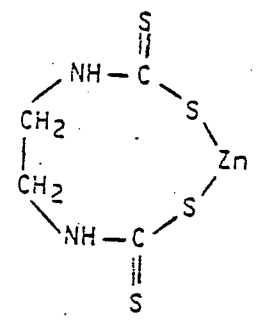

(A)

Zink-ethylen-1,2-bis-(dithiocarbamat)

## Beispiel A

Venturia-Test (Apfel) / protektiv

Lösungsmittel: 4,7 Gew.-Teile Aceton
Emulgator: 0,3 Gew.-Teile Alkyl-aryl-polyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknung des Spritzbelages werden

die Pflanzen mit einer wäßrigen Konidiensuspension des Apfelschorferregers (Venturia inaequalis) inokuliert und verbleiben dann einen Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 1, 2, 11, 21.

Beispiel B

Erysiphe-Test (Gerste) / protektiv

Lösungsmittel: 100 Gew.-Teile Dimethylformamid
Emulgator: 0,25 Gew.-Teile Alkylarylpolyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknung des Spritzbelages werden die Pflanzen mit Sporen von Erysiphe graminis f. sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigt bei diesem Test z.B. die Verbindung gemäß folgendem Herstellungsbeispiel: 18.

Patentansprüche

1. Heteroarylthiovinyl-Verbindungen der Formel (I)

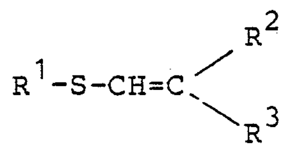

(I),

in welcher

$R^1$ für einen gegebenenfalls substituierten heteroaromatischen Rest steht und

$R^2$ und $R^3$ gleich oder verschieden sind und für Cyano, Nitro, Alkoxycarbonyl, Alkylsulfonyl oder für gegebenenfalls substituiertes Phenylsulfonyl stehen.

2. Heteroarylthiovinyl-Verbindungen gemäß Anspruch 1, wobei in Formel (I)

$R^1$ für einen gegebenenfalls ein- oder zweifach, gleich oder verschieden substituierten, über Kohlenstoff verknüpften heteroaromatischen 5- oder 6-gliedrigen Ring steht, der benzoanneliert sein kann. Als Substituenten seien Alkyl mit 1 bis 4 Kohlenstoffatomen, Halogen und Nitro genannt,

$R^2$ und $R^3$ gleich oder verschieden sind und für Cyano, Nitro, Alkoxycarbonyl oder Alkylsulfonyl mit je 1 bis 4 Kohlenstoffatomen im Alkylteil oder für gegebenenfalls ein- oder zweifach, gleich oder verschieden substituiertes Phenylsulfonyl stehen. Als Substituenten seien Alkyl mit 1 bis 4 Kohlenstoffatomen und Halogen genannt.

3. Heteroarylthiovinyl-Verbindungen gemäß Anspruch 1, wobei in der Formel (I)

$R^1$ für über Kohlenstoff verknüpftes, gegebenenfalls ein- oder zweifach gleich oder verschieden substituiertes Pyridyl, Pyrimidyl, Imidazolyl, Chinolyl, Benzthiazolyl, Benzimidazolyl und Benzoxazolyl steht, wobei als Substituenten vorzugsweise genannt seien: Methyl, Ethyl, n-Propyl, iso-Propyl, n-, iso-, sec- und t-Butyl, Chlor, Brom und Nitro,

$R^2$ und $R^3$ gleich oder verschieden sind und für Cyano, Nitro, Methoxycarbonyl, Ethoxycarbonyl, Methylsulfonyl, Ethylsulfonyl und für gegebenenfalls ein- oder zweifach, gleich oder verschieden substituiertes Phenylsulfonyl stehen, wobei als Substituenten Methyl, Ethyl, Chlor und Brom genannt seien.

4. Verfahren zur Herstellung von Heteroarylthiovinyl-Verbindungen der Formel (I)

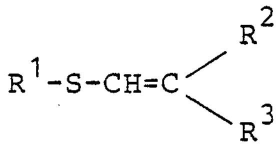

(I)

in welcher

$R^1$ für einen gegebenenfalls substituierten heteroaromatischen Rest steht und

$R^2$ und $R^3$ gleich oder verschieden sind und für Cyano, Nitro, Alkoxycarbonyl, Alkylsulfonyl oder für gegebenenfalls substituiertes Phenylsulfonyl stehen,

dadurch gekennzeichnet, daß man substituierte Vinylhalogenide der allgemeinen Formel (II)

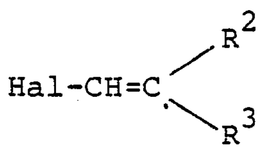

(II)

in welcher

$R^2$ und $R^3$ gleich oder verschieden sind und für Cyano, Nitro, Alkoxycarbonyl, Alkylsulfonyl oder für gegebenenfalls substituiertes Phenylsulfonyl und

Hal für Halogen stehen,

mit Heteroarylthiolen der Formel (III)

$$R^1\text{-SH} \qquad (III)$$

in welcher

$R^1$ für einen gegebenenfalls substituierten heteroaromatischen Rest steht,

in Gegenwart eines organischen Verdünnungsmittels und in Gegenwart einer Base umsetzt.

5. Schädlingsbekämpfungsmittel, gekennzeichnet durch einen Gehalt an mindestens einer Heteroarylthiovinyl-Verbindung der Formel (I).

6. Verwendung von Heteroarylthiovinyl-Verbindungen der Formel (I) zur Bekämpfung von Schädlingen.

7. Verfahren zur Bekämpfung von Schädlingen, dadurch gekennzeichnet, daß man Heteroarylthiovinyl-Verbindungen der Formel (I) auf Schädlinge und/oder ihren Lebensraum einwirken läßt.

8. Verfahren zur Herstellung von Schädlingsbekämpfungsmitteln, dadurch gekennzeichnet, daß man Heteroarylthiovinyl-Verbindungen der Formel (I) mit Streckmitteln und/oder oberflächenaktiven Mitteln vermischt.

Europäisches Patentamt

# EUROPÄISCHER RECHERCHENBERICHT

0093908

Nummer der Anmeldung

EP 83 10 3892

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | US-A-3 136 689 (MONSANTO COMPANY)<br>* Insgesamt *<br>--- | 1-5 | C 07 D 213/70<br>C 07 D 277/74<br>C 07 D 239/38<br>C 07 D 215/36<br>C 07 D 233/84<br>C 07 D 235/28<br>C 07 D 263/58<br>A 01 N 43/00 |
| A | US-A-4 282 231 (STAUFFER CHEMICAL COMPANY)<br>* Ansprüche *<br>--- | 1,5 | |
| A | EP-A-0 001 312 (STAUFFER CHEMICAL COMPANY)<br>* Beispiel 5; Seite 8; Ansprüche *<br>----- | 1,5 | |
| | | | RECHERCHIERTE SACHGEBIETE (Int. Cl. 3) |
| | | | C 07 D 213/00<br>C 07 D 277/00<br>C 07 D 239/00<br>C 07 D 215/00<br>C 07 D 233/00<br>C 07 D 235/00<br>C 07 D 263/00 |

Der vorliegende Recherchenbericht wurde für alle Patentansprüche erstellt.

| Recherchenort | Abschlußdatum der Recherche | Prüfer |
|---|---|---|
| DEN HAAG | 03-08-1983 | VAN BIJLEN H. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82